Europäisches Patentamt

European Patent Office

Office européen des brevets

(11) Publication number: **0 316 023 B1**

## EUROPEAN PATENT SPECIFICATION

(45) Date of publication of patent specification: **09.06.93**

(51) Int. Cl.5: **C12N 15/70**, C12N 1/21, C12P 21/00

(21) Application number: **88121510.7**

(22) Date of filing: **07.03.84**

(60) Publication number of the earlier application in accordance with Art.76 EPC: **0 121 138**

(54) **Plasmids, methods for their construction, microorganisms carrying them and methods for the extracellular production of xylanase by cultivation of the microorganisms.**

(30) Priority: **08.03.83 JP 38087/83**
**08.03.83 JP 38089/83**
**09.12.83 JP 232507/83**
**09.12.83 JP 232508/83**

(43) Date of publication of application:
**17.05.89 Bulletin 89/20**

(45) Publication of the grant of the patent:
**09.06.93 Bulletin 93/23**

(84) Designated Contracting States:
**AT BE CH DE FR GB IT LI NL SE**

(56) References cited:
**GB-A- 2 091 268**

**AGR. BIOL. CHEM., vol. 37, no. 9, 1973, pages 2097-2103; K. HORIKOSHI et al.: "Xylanase produced by alkalophilic Bacillus No. C-59-2"**

**GENE, vol. 26, 1983, pages 59-65, Elsevier Science Publishers; R. BERNIER et al.: "Molecular cloning of a Bacillus subtilis xylanase gene in Escherichia coli"**

(73) Proprietor: **RIKAGAKU KENKYUSHO**
**2-1 Hirosawa**
**Wako-shi Saitama-ken(JP)**

(72) Inventor: **Horikoshi, Koki**
**No.4-39-8, Sakuradai Nerima-ku**
**Tokyo(JP)**
Inventor: **Kudo, Toshiaki**
**No.1-21-20-606, Taira-cho Meguro-ku**
**Tokyo(JP)**
Inventor: **Kato, Chiaki**
**Nisshinjyutaku 1-307 No. 2-1607-2, Nisshin-cho**
**Ohmiya-shi Saitama-ken(JP)**
Inventor: **Honda, Hiroshi**
**No. 1-1-1, Hinodai**
**Hino-shi Tokyo(JP)**

(74) Representative: **Vossius & Partner**
**Siebertstrasse 4 P.O. Box 86 07 67**
**W-8000 München 86 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services
(3. 10/3.6/3.3. 1)

## Description

This invention relates to novel plasmids, methods for their construction, transformed microorganisms carrying them and to methods for the extracellular production of xylanase by cultivation of the microorganisms. The novel plasmids carry DNA sequences encoding the enzyme xylanase and are capable of inducing the extracellular secretion of the high-molecular xylanase by corresponding transformed microorganisms.

Plasmids are non-chromosomal elements of circular DNA found in a microorganism cell. Plasmids are currently being used as a means for recombination of microorganism genes and are becoming more and more important in the field of fermentation industry.

Studies have recently been effected on plasmids carrying a foreign DNA which carries the genetic information for metabolic products of specific demand for the growth of microorganisms, as shown in the production of amino acids or peptides. Some plasmids have been introduced into host microorganisms to obtain transformants. Methods have been proposed for producing relatively low molecular compounds such as amino acids and peptides by cultivating the transformants. However, the degree of propagation of plasmids carrying genes for production of high-molecular substances depends on the nature of the host microorganisms and those plasmids have not effectively been expressed. Furthermore, no effective methods for the cultivation of such transformants have been established.

It has not been possible to selectively obtain a certain extracellular high-molecular product by cultivating a microorganism transformed with a plasmid carrying a foreign DNA fragment carrying the genetic information for the extracellular production of high-molecular substances which are metabolic products of other microorganisms. Such extracellular production has not successfully been done even when a transformant of Escherichia species, which is usually used as a host microorganism, is used.

US Patent 4,411,994 of W.Gilbert et al discloses a process for producing specific proteins in bacteria and having them excreted from the bacterial cell. This process comprises inserting the DNA coding for the desired non-bacterial protein or part of a protein by recombinant techniques into a plasmid or phage gene which codes for either a periplasmic or an extracellular protein, hereinafter called a "carrier protein", then transforming a bacterial host with the recombinant DNA and culturing the transformed host to excrete the protein. The process of this patent provides a means for producing a selected protein by employing a gene for a carrier protein which has a leader sequence of hydrophobic amino acids at its amino terminus.

The cell wall of Escherichia coli which has often been used for the production of useful physiologically active substances, consists of three kinds of membranes: an inner membrane, peptidoglycan and an outer membrane. The space between the inner and outer membrane is called the periplasmic space. The process of US Patent 4,411,994 permitted the excretion of the products into the periplasmic space but not into the culture medium through the outer membrane or outside of the bacterial host cell. The present invention permits the excretion of xylanase through the outer membrane of the host microorganism and its direct recovery from the culture medium by insertion of a DNA sequence carrying the genetic information for the extracellular production of the enzyme xylanase, a high-molecular substance, into a plasmid, whereby a hybrid plasmid is obtained and by culturing the host microorganism transformed with said hybrid plasmid. Thus, the present invention permits the mass production of the enzyme xylanase which has never been possible by prior art processes.

Therefore, the technical problem underlying the present invention is to provide novel plasmids containing a DNA sequence carrying the genetic information for the extracellular production of xylanase, methods for their construction, transformed microorganisms carrying the plasmids and to provide methods for the cultivation of the microorganisms for the extracellular production of xylanase. The plasmids of the present invention are constructed from a vector plasmid and a DNA fragment carrying the genetic information for the extracellular production of xylanase. Such DNA fragments are obtained from microorganisms of the genus Bacillus. The term "extracellular production" means that the xylanase produced by the host microorganism is secreted into the culture medium.

The recombinant plasmids of the present invention which are capable of inducing the extracellular secretion of xylanase in bacteria of the genus Escherichia are obtainable by carrying out the following steps:

(a) preparing with a restriction enzyme, preferably with Hind III, a chromosomal DNA fragment of Bacillus sp. C 125 (FERM BP-469) which codes for the extracellular secretion of xylanase;

(b) preparing the recombinant plasmid DNA by ligating the chromosomal DNA fragment obtained in step (a) into a vector plasmid which was digested with a suitable restriction enzyme, preferably Hind III

(d) isolating the recombinant plasmid DNA from a transformed host cell which exhibits a xylanase-producing phenotype.

The transformed host microorganisms of the present invention belong to the genus Escherichia. They are capable of of secreting the xylanase encoded by the incorporated plasmid of the present invention.

The present invention also provides a method for cultivation of the transformed host microorganisms, which comprises inoculating the microorganism into a culture medium containing an inorganic salt necessary for the microorganism to grow, or in a culture medium containing a selected carbon source together with the inorganic salt, keeping on culturing the microorganism after the concentration of the microorganism cells has reached the maximum and until the production and accumulation of the xylanase in the media reached the maximum.

Brief Description of the Drawings

Fig.1 and Fig.2 show bacterial growth and xylanase production by Escherichia coli HB101(pCX311) of the present invention.

Fig.3 shows xylanase production by Bacillus sp. C125.

Fig.4 shows the restriction enzyme cleavage map for the plasmid pCX311.

Fig.5 shows the identity of pH activity of xylanase produced by Escherichia coli HB101(pCX311) with that produced by Bacillus sp. C125.

The term "high molecular substances" used in the specification and the claims of this application means useful physiologically active compounds including bacterial metabolic products such as enzyme proteins, antibiotics, and biologically active proteins of mammalian origin.

The present invention will now be explained in detail.

(a) The Plasmids and their Construction

The novel plasmids of the present invention are constructed by inserting a foreign DNA into a plasmid (extrachromosomal DNA or vector DNA), such as Col $E_1$, found in Escherichia cells.

The vector DNA which can be used in the present invention may have been isolated from natural sources.

The DNA fragments unnecessary for self-reproduction may have been deleted. Specific examples for vectors which may be used are the plasmids $ColE_1$, pMB9, pBR322, pSC101, R6K and lambda phage.

Foreign DNA fragments or exogenates which can be inserted into the vector DNA are the genes having genetic information of extracellular production or secretion of high-molecular substances, such as enzyme proteins, for example, xylanase, penicillinase, alkaline phosphatase, $\beta$-galactosidase, amylase, protease, $\beta$-glucanase, cellulase.

Exogenates or DNA fragments which can be used in the present invention include the genes having genetic information of extracellular production or secretion of high molecular substances. They are obtained from a microorganism which belongs to, for example, the genus Bacillus.

An example of a microorganism containing DNA which has genetic information of extracellular xylanase production and which can be used in the present invention is Bacillus sp. C125.

Bacillus sp. C125 was isolated from a soil sample collected in Tsurugashima, Saitama, Japan and shows the following characteristic microbiological properties. Examination and classification of this strain were done according to "Aerobic Spore-forming Bacteria" (United State Department of Agriculture, Nov. 1952 by N.R. Smith, R.E. Gordon & F.E. Clark) and "Bergey's Manual of Determinative Bacteriology (1957)-".

Characteristic Microbiological Properties:

a) Morphology

1) Rods, 0.5 - 0.7$\mu$ x 3.0 - 4.0$\mu$

2) the spore-forming oval, 0.6-0.8$\mu$ x 1.0-1.2$\mu$ and the sporangium is swollen.

3) Gram positive.

b) Growth on Culture Medium

| Culture Medium | Growth | |
|---|---|---|
| | pH7 | pH10 |
| 1) Bouillon | growing | good |
| 2) Bouillon-agar | " | " |
| 3) Glucose-bouillon | slightly growing | " |
| 4) Glucose-bouillon-agar | " | " |
| 5) Tyrosine-agar | " | " |
| 6) Medium-I | " | " |
| 7) Glucose-nitrate | growing | " |
| 8) Glucose-asparagine-agar | nil | " |
| 9) Medium-I + 5%NaCl | growing | " |

c) Physiological Properties

| | pH7 | pH10 |
|---|---|---|
| 1) Nitrate reduction | no | almost nil |
| 2) Utilization of propionate | no | yes |
| 3) VP test | negative | negative |
| 4) Urease test | negative | neutral |
| 5) Hydrolysis of starch | positive | positive |
| 6) Hydrolysis of casein | positive | positive |
| 7) Growth condition | grows below pH 11.0 and below 55°C | |
| 8) Growth under no-oxygen condition (on Medium-I) | no | slightly growing |

Summarizing the properties above, it is clear that the microorganism belongs to the genus Bacillus because it is an aerobic, spore-forming bacterium. The microorganism Bacillus sp. C125 is a new strain of alkalophilic bacteria. It is clearly different from known bacteria in that it grows at a pH lower than 11.0 and at a temperature lower than 55°C.

For the purpose of the insertion of foreign DNA into a vector DNA, any conventional method can be used in the present invention. For instance, chromosomal DNA is digested with a suitable restriction enzyme or endonuclease to obtain a foreign DNA fragment or an exogenote which is then mixed with a vector DNA which has been treated with the same restriction enzyme, and the mixture is ligated by a suitable ligase. Thus, as described before, the novel plasmids of the present invention can be obtained by preparing, with a restriction enzyme, a DNA fragment coding for the extracellular production of xylanase, digesting a plasmid vector DNA with a restriction enzyme which does not interfere with the genetic information carried on the DNA fragment, treating the DNA fragment and the digested vector plasmid with DNA ligase to construct a recombinant plasmid and isolating the recombinant plasmid by a conventional manner [see Bolivar et. al., Gene, 2, 95 (1977)].

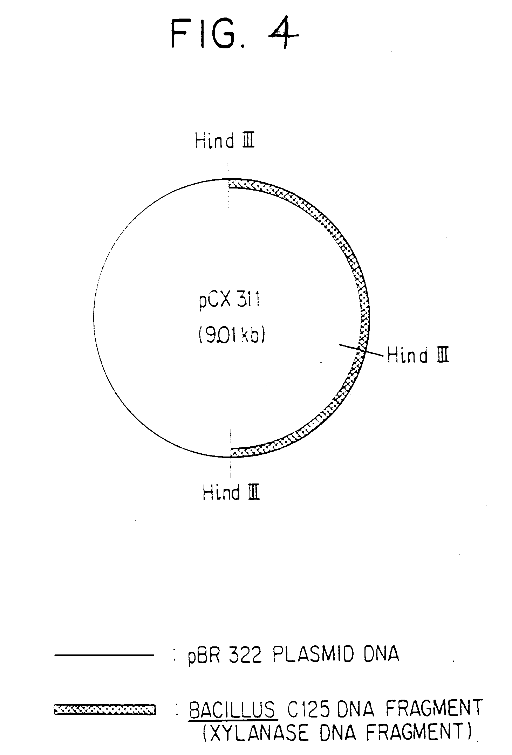

The novel plasmid pCX311 can be constructed from a fragment of Bacillus sp. C125 chromosomal DNA and plasmid pBR322. The restriction enzyme map for plasmid pCX311 is shown in Fig. 4. As seen in Fig. 4, plasmid pCX311 is constructed from plasmid pBR322 into which the fragment of Bacillus sp. C125 chromosomal DNA coding for the extracellular xylanase production is inserted at the Hind III restriction site of plasmid pBR322. Thus, plasmid pCX311 is a cyclic DNA molecule of about 9.01 kb, consisting of pBR322 DNA and a fragment of Bacillus sp. C125 DNA of about 4.0 kb.

(b) Preparation of Microorganisms

The recombinant plasmids constructed from the chromosomal DNA fragments and the vector DNA plasmids are introduced into a host microorganism of the genus Escherichia by a conventional transforma-

EP 0 316 023 B1

tion technique. Cultivation is kept on until a stabilized genotype is established, to obtain a transformant carrying both genotypes on the selected chromosomal DNA and on the vector DNA. For this purpose, a conventional, so called shot gun method can be used.

A typical host microorganism which can be used in the present invention is Escherichia coli HB101 which is a hybrid strain of Escherichia coli K-12 and Escherichia coli B.

The transformant, Escherichia coli HB101 (pCX311) which is prepared by introducing plasmid pCX311 into Escherichia coli HB 101 has the same microbiological properties as the host, Escherichia coli HB101, except penicillin resistance and xylanase production [Molecular Cloning, A Laboratory Manual, p.504 (1982), Genotype: F⁻, hsd S 20($r_B^-$ , $m_B^-$ ), rec A13, ara-14, proA2, lacY1, galK2, rpsL20(Sm'), xyl-5 mtl-1, supE44λ⁻]. Escherichia coli HB101 (pCX311) is further characterized by the property of plasmid pCX311, that is, the capability of extracellular production of xylanase.

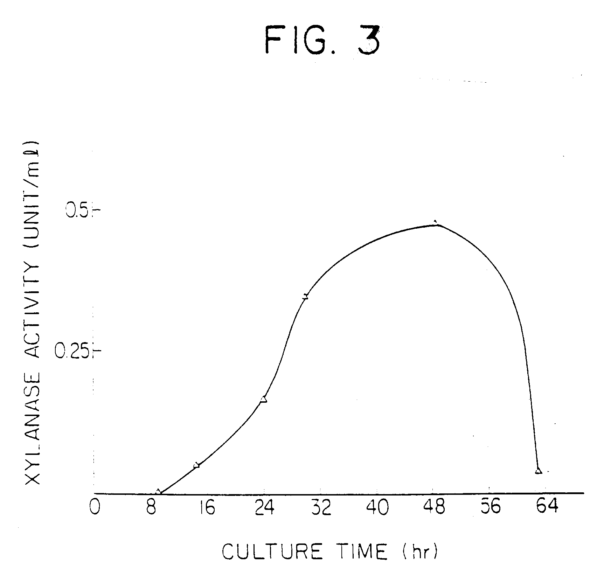

The extracellular production of xylanase by Escherichia coli HB101 (pCX311), as shown in the Examples, reaches more than 80% of total production including the intracellular production and remains after long cultivation. By contrast, extracellular production or activity of xylanase of Bacillus sp. C125, as shown in Fig. 3, reaches the maximum slowly and then, it drops rapidly. Thus, the extracellular production of Bacillus sp. C125 can not be compared with that of Escherichia coli HB101 (pCX311) of the present invention.

The present invention which provides Escherichia coli HB 101 (pCX311) having the capability of extracellular production of enzyme proteins such as xylanase is not only novel but also inventive. E. coli HB101 (pCX311) can produce and secrete into the medium from the cells, in addition to large amounts of xylanase, large amounts of other enzyme proteins which can also be collected. More specifically, it has been discovered that Escherichia coli HB 101 (pCX311) produces and secretes into the medium from the cells, as shown in the Examples, large amounts of penicillinase, alkaline phosphatase, in addition to xylanase which have ever been observed only in the periplasmic space.

Such extracellular production by Escherichia coli HB101 (pCX311) demonstrates that the DNA of about 4000 nucleotide base pairs carried on plasmid pCX311 provides the host with the capability of extracellular production of the metabolic products.

(c) Cultivation of the microorganisms

For culturing the transformants prepared in step (b), any culture media can be used which are suitable for the production of specific substances for which specific genetic information codes and which are suitable for growth of the microorganisms of the genus Escherichia. In the process of the present invention, it is necessary to culture the microorganism in a culture medium containing an inorganic salt necessary for the microorganism to grow, or a selected carbon source together with the inorganic salt, and to keep on culturing the microorganism, after the concentration of the microorganism cells reached the maximum, and until the production and accumulation of the high-molecular substances in the media reach the maximum.

Examples of the inorganic salt which can be used in the present invention include sodium and potassium salts such as sodium chloride, sodium sulfate, potassium chloride and the like, among which sodium chloride is preferred. The media containing the inorganic salt or the selected carbon source together with the inorganic salt can contain such carbon sources as glucose, sucrose, lactose, maltose, glycerol, bran, xylan and the like, such nitrogen sources as ammonia water, ammonium salts, inorganic ions and optionally such nutrients as amino acids, vitamins. In the culture of Escherichia coli HB101 (pCX311), it is important and necessary to add to the medium the carbon source suitable for a selected product. The culture media which can be used in the present invention are those which use, as a basic medium, LB-broth (containing tryptone, yeast extract and NaCl), BPB-broth (Difco Laboratories; containing polypeptone, yeast extract and $K_2HPO_4$), nutrient broth (Difco 0001), tryptone-NaCl broth.

Since more than 80% of total products remain in the intracellular fraction in a medium containing no inorganic salt, it is necessary to use a medium containing an inorganic salt in order that most products are secreted into the culture medium out of the cells. The amount of inorganic salt used is in the range of 0.5 - 3.0% by weight on the basis of the culture medium.

In the culture of Escherichia coli HB101 (pCX311), excellent results are obtained by the use of LB-broth to which bran and/or xylan have been added as carbon sources, preferably in the amount each of 0.5% by weight on the basis of the culture medium.

Although culture conditions such as pH, temperature or oxygen supply can be changed for the optimum growth of the microorganism of the genus Escherichia, it is necessary, in the present invention, to keep on culturing the microorganism, after the concentration of the microorganism cells reached the maximum, that is to say, after the latter logarithmic phase and until the production and accumulation of the high-molecular

5

substances in the medium reach the maximum.

After the inoculation of the microorganism of the genus Escherichia in the medium, the cell concentration reaches the maximum after 5 to 20 hours and the production and accumulation of the high-molecular substances reach the maximum after 12 to 48 hours. Although the pH of the culture medium is not critical, it is preferred in the range of pH5 - pH8, especially pH7. Thus, without further addition of inorganic salt necessary for the microorganism to grow, carbon sources and the like during cultivation, the microorganism produces large amounts of extracellular high-molecular substances which can conveniently be collected.

According to the culture method of the present invention, in addition to enzyme proteins, such high-molecular fermentation products as antibiotics, polysaccharides can be produced in a significant amount. This method can be applied to the cultivation of any microorganisms transformed with a plasmid carrying a foreign DNA coding for biologically active high-molecular substances such as hormone peptides (e.g. insulin) or interferon and the DNA coding for extracellular production or secretion of biologically active high-molecular substances, which leads to mass production of the high-molecular substances such as insulin, interferon and the like.

As explaind above, the present invention contributes to the industrial production of useful high-molecular substances.

Examples of microorganisms which may be used in this invention include (i) Bacillus sp. C125 and (ii) Escherichia coli HB101 (pCX311), all of which were deposited with the Fermentation Research Institute, Agency of Industrial Science and Technology, Ministry of International Trade and Industry, Japan, International Depository Authority (hereinafter referred to as "FERM") under the following accession numbers, respectively, FERM BP-467, FERM BP-468,FERM BP-469 and FERM BP-470 and are on deposit with FERM in an unrestricted deposit permitting the full access to the culture.

The depository and accession numbers of the above mentioned microorganisms are shown below:

| Microorganims | Depository | Accession No. |
|---|---|---|
| (i) Bacillus sp. C125 | FERM | FERM BP-469 |
| (ii) Escherichia coli HB101 (pCX311) | FERM | FERM BP-470 |

The applicant will maintain the deposition of FERM BP-469 and FERM BP-470 in an unrestricted form until the end of the duration of a patent granted on this application if a patent is granted on this application, and thus said microorganism strains will be available to any third party at any time until the end of the duration of a patent granted on this application.

We will now explain by reference to Examples methods for the construction of the plasmids of the present invention, methods for preparation of the transformant Escherichia coli HB101 (pCX311), and the extracellular production of xylanase by the transformants.

Example 2

Preparation and purification of plasmid pCX311.

6

1. Culture growth of bacterial cells using <u>Escherichia coli</u> HB101 (pCX311) [FERM BP-470]

```
Preculture in LB.      Over night at 37°C.
     ↓
Culture in M-9.        Add chloramphenicol after 130 Klett
     ↓                 units.  16hr at 37°C.

Centrifugation.        5000rpm,20min at 4°C.
     ↓
Cell pellets.          Stored at -30°C.
```

2. Purification of plasmid DNA.

```
Cell pellets

     |   10ml, 20%Sucrose, 50mMTris, 1mMEDTA PH8.
     |   Suspended, Keep on ice.
     ↓
50ml polypropylene centrifuge tube.

     |   2ml, 0.25M EDTA.
     |   1ml, Lysozyme (5mg/ml of 0.025M Tris, PH8.)
     |   0.1ml, RNases (10mg/ml)
     ↓
Cell lysis.  Mix gently.  Stand 15 to 30 min on ice.

     |   5ml, 3x Triton©  Mix gently.  Stand 15 to 45 min on ice.
     ↓
Centrifugation   17,000rpm, 40min at 4°C.
     ↓
Supernatant soln in plastic cylinder.
     ↓
250ml glass bottle.

     |   2/3 vol DD H₂O
     |   2/3 vol Cold saturated phenol.  Mix gently
     ↓
Centrifugation.  6500rpm, 15min at 4°C.
     ↓
Upper phase.

     |   Equal vol. of phenol; Chloroform.
     ↓
Centrifugation.  6500rpm, 15min at 4°C.
     |
```

Upper phase in 250ml bottle.
  1/25 vol 5M NaCl.
  2 vol EtOH at -20°C.
        Over night at -20°C.

Centrifugation.  6500rpm, 60min at -20°C.

DNA pellet. Dry excess liquid.

  5ml A-50 buffer, resuspended.
  1ml sterile 80% glycerol, mix gentry.

A-50 column. (2x35cm,1fraction=4ml.)

DNA fraction. ($A_{260}$ peak.)
  2 vol EtOH at -20°C.  Over night at -20°C.

Centrifugation.  6500rpm, 60min at -20°C.

DNA pellet.

  2.1ml TEN buffer in 5ml cellulose nitrate tube.
  2.2g CsCl, mix.

  (In dark.)

  150ul PdI(2mg/ml).  Mix well.
  2ml mineral oil on top of tube.

CsCl gradient centrifugation.  36,000rpm,40hr at 20°C.
                    Upper band; chromosomal & nicked
  Visible with UV.  DNA.
                    Lower band; covalently closed
                    p-DNA.

Collect of lower band DNA by drop wise.

Dowex 50W-X8 column.  UV check.

  (In light.)

Dialysis against 2/41 of 10mM Tris, 1mM EDTA pH8.
                    Over night at 4°C.

In 30ml cortex centrifuge tube.          vol.

  1/25 vol 5M NaCl.
  2 vol EtOH at -20°C.
                    Over night at -20°C.

Centrifugation.  6500rpm,60min at -20°C.

DNA precipitates.

  1-2ml TEN buffer.

Purified plasmid DNA (1mg/1-broth)  Stored at -20 to 70°C.

Example 1

(1) Preparation of a chromosomal DNA having genetic information for the production of xylanase.

Alkalophilic Bacillus sp. C125 (FERM BP-469) having the capability of extracellular production of xylanase was cultured with shaking at 30°C for 19 hours in the broth (containing 10.0g of bran, 5.0g of yeast extract, 5.0g of polypeptone, 0.2g of $MgSO_4 \cdot 7H_2O$, and 10g of $Na_2CO_3$ in one liter of water and adjusted to pH 9.0). The cells in the latter logarithmic phase were collected, from which a chromosomal DNA was extracted by the phenol extraction method and purified to obtain 5mg of the chromosomal DNA.

(2) Insertion of chromosomal DNA fragment into vector DNA.

The chromosomal DNA (10μg) obtained in step (1) was digested with the restriction enzyme Hind III at 37°C for 5, 10, 20, 30 and 60 minutes to obtain DNA fragments.

Plasmid pBR322 [Bethesda Research Laboratories, U.S.A., tetracyclin resistant ($Tet^r$) and ampicillin resistant (Amp $^r$)] used as the vector was cut with Hind III, then heated at 65°C for 5 minutes, and then mixed with the DNA fragments. The mixture was treated with $T_4$ phage DNA ligase at 10°C for 24 hours and then heated at 65°C for 5 minutes.

Three times volume of ethanol was added to the mixture. Plasmids carrying the chromosomal DNA fragments were precipitated and collected.

(3) Transformation of the microorganism with plasmids having gene for extracellular production of xylanase.

Escherichia coli HB101 which is a hybrid strain of Escherichia coli K-12 and Escherichia coli B, was inoculated in 10ml of LB-broth [containing 10g of tryptone (Difco Laboratories, Detroit, Mich.), 5g of yeast extract, 1g of glucose and 10g of NaCl in one liter of deionized water; pH was adjusted to 7.0] and cultured at 37°C with shaking until the latter logarithmic phase. The cells were collected and suspended in an ice-cold $CaCl_2$ solution of 0.03M (final concentration) to obtain competent cells. The cell suspension and the plasmid solution obtained in step (2) were combined and kept on ice for 60 minutes. The mixture was heated to 42°C for 1 to 2 minutes to introduce the plasmid DNA into the cells. This cell suspension was inoculated in fresh LB-broth and cultured at 37°C for 3 to 5 hours with shaking. The cells were collected and washed to obtain transformants, from which Escherichia coli HB101 (pCX311) (FERM BP-470) having extracellular producibility of xylanase and penicillinase was isolated.

Example 2 : see above

Example 3

Escherichia coli HB101 (pCX311) (FERM BP-470) obtained in step (3) of Example 1 was inoculated in 500ml-flasks containing 100ml of LB-broth (containing 10g of tryptone, 5g of yeast extract, 1g of glucose, 2g of glycerol, 10g of NaCl and 10mg of penicillin in one liter of water) containing 0.5% xylan and cultured at 37°C with shaking. Cell growth was measured by optical density at 660nm. Enzyme activity was assayed as follows: 0.05ml of the culture fluid, 0.1ml of xylan solution (Seikagaku Kogyo, Japan) and 0.1ml of 0.2M Tris-malate buffer of pH8.0 were mixed and heated at 40°C for 10 minutes. One ml of DNS (3.5-dinitrosalicylic acid) was added to the mixture, which was then heated at 100°C for 5 minutes. Four ml of water was added to the mixture. Absorbance at 510nm was measured. One unit of xylanase reduces one mg of xylose per one minute.

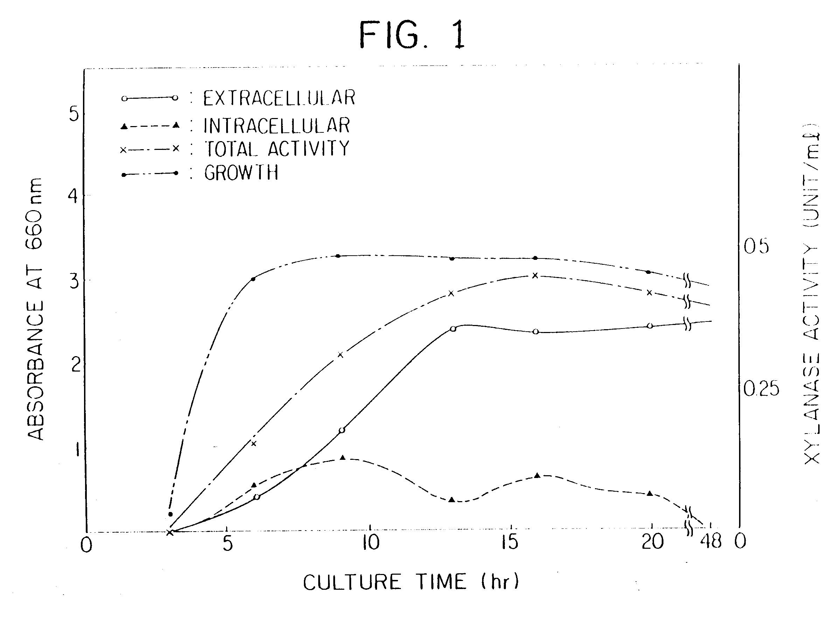

Fig. 1 shows extracellular production or secretion of xylanase by Escherichia coli HB101 (pCX311) cultured in the LB-broth containing 0.5% xylan.

As shown in fig. 1, cell growth of the transformant reached the maximum after 9 to 12 hours cultivation and extracellular xylanase activity began to increase after 6 hours and reached the maximum (about 0.35 unit/ml) after 13 hours cultivation.

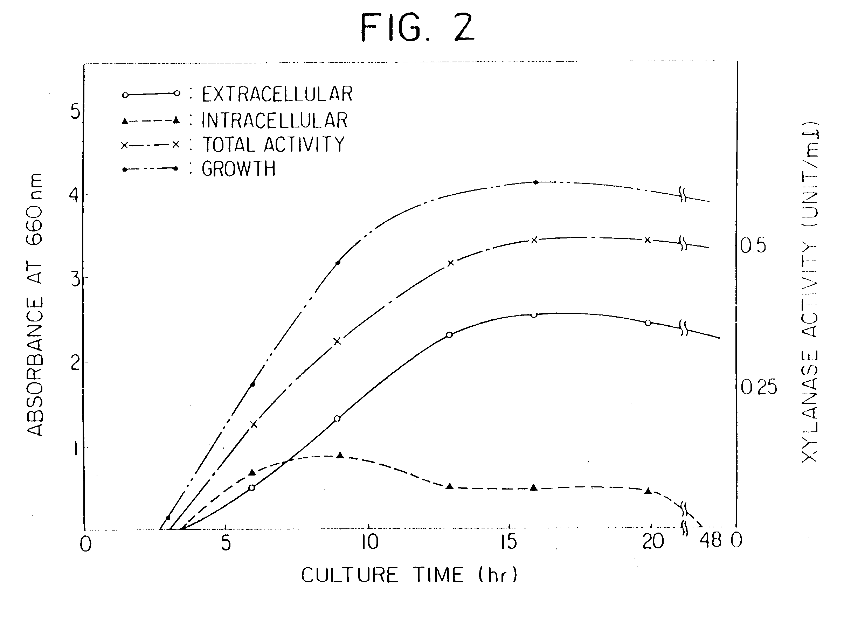

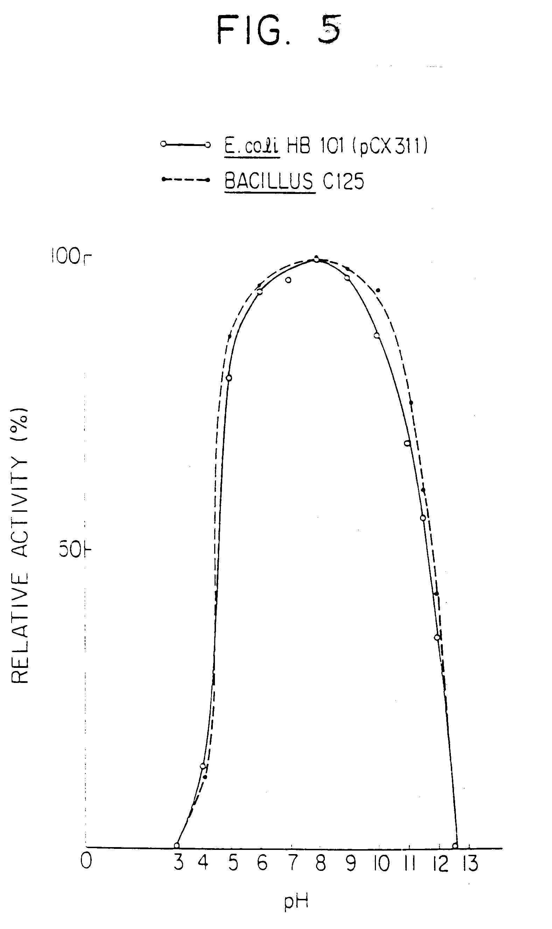

Extracellular xylanase produced was very stable and, as shown in fig. 5, the production was kept high even after 48 hours cultivation and reached more than 80% of total enzyme production. In contrast, intracellular xylanase production was detected a little at an early phase (after 9 hours cultivation). But, the maximum was only 0.13 unit/ml or about 10% of total enzyme production and furthermore, the intracellular production decreased gradually. Fig. 2 shows xylanase production by Escherichia coli HB101 (pCX311) cultured in the LB-broth containing 0.5% bran instead of xylan. Fig. 2 shows the similar tendency as observed in Fig. 1. As a comparison, Bacillus sp. C125 (FERM BP-469) which is a DNA donor was cultured and xylanase activity was assayed.

Bacillus sp. C125 was inoculated in 500ml-flasks containing 100ml of the medium (which contained 10.0g of xylan, 5.0g of yeast extract, 5.0g of polypeptone, 1.0g of $K_2HPO_4$, 0.2g of $MgSO_4 \cdot 7H_2O$ and 10g of $Na_2CO_3$ in one liter of water and was adjusted to pH 6.0) and cultured at 37°C with shaking. Extracellular xylanase activity in the culture fluid was observed every eight hours. It began to increase after eight hours cultivation and reached the maximum (about 0.5 unit/ml) after 48 hours, but it decreased quickly (Fig. 3).

[Identification of xylanase]

Ammonium sulfate was added to the culture fluid of the transformant obtained in step (3) of Example 1. The precipitate salted out was dissolved in water and the solution was dialyzed overnight against running water. The dialyzate was adsorbed on a CM-cellulose column equilibrated with 20mM phosphoric acid-monosodium phosphate buffer of pH 4.5. Elution was done by applying a linear gradient of 0.1-0.7M sodium chloride. Xylanase was eluted with about 0.4M sodium chloride. The fractions exhibited xylanase activity were combined and applied on a Sephadex G-100 gel filtration to obtain purified xylanase.

Similarly, the culture fluid of Bacillus sp. C125 (FERM BP-469) was treated to obtain purified xylanase.

For the identification of Escherichia coli HB101 (pCX311) xylanase with Bacillus sp. C125 xylanase, effects of pH on activity, ultracentrifugal analysis, electrophoresis and estimation of molecular weight of each xylanase were examined. As a result, both the enzymes were identical with each other as shown below.

(a) Acetate (pH 4 - 5), Tris-malate (pH 5 - 8), Tris-HCl (pH 7 -9), and glycine-NaOH (pH 9 - 11) were prepared. Using these buffer solutions, effects of pH on activity was examined. Results are shown in Fig. 5 which exhibits the identification of both the enzymes on pH activity.

(b) Ultracentrifugal analysis showed that both the enzymes had single peak of about 3.5S of sedimentation coefficient.

(c) Desk electrophoresis at pH 8.3 showed that both the enzymes had single band. Electrofocusing by Ampholine showed single peak. Isoelectric point of both the enzymes was pH6.3.

(d) Estimation of molecular weight of the enzymes was done by SDS-polyacrylamide method. Estimated molecular weight of both the enzymes was about 40,000.

Example 4

Escherichia coli HB101, Escherichia coli HB101 (pBR322) and Escherichia coli HB101 (pCX311) (FERM BP-470) were cultured in the same, xylan-containing LB-broth as used in Example 3 at 37°C with shaking for 20 hours (penicillinase) or for 15 hours (alkaline phosphatase and $\beta$-galactosidase). Enzyme activities of alkaline phosphatase and $\beta$-galactosidase were measured by optical density at 420nm. Results are given in Table 1.

Table 1

| Microorganism | Products | Activity (U/ml) | | |
|---|---|---|---|---|
| | | Extracellular | Intracellular | Total |
| E. coli HB101 | Alkaline phosphatase | 0.02 (2%) | 1.31 (98%) | 1.33 (100%) |
| | $\beta$-Galactosidase | 0.03 (2%) | 1.20 (98%) | 1.23 (100%) |
| E. coli HB101 (pBR322) | Alkaline phosphatase | 0.01 (2%) | 0.47 (98%) | 0.48 (100%) |
| | $\beta$-Galactosidase | 0.00 (0%) | 0.80 (100%) | 0.80 (100%) |
| | Penicillinase | 0.20 (3%) | 9.52 (97%) | 9.72 (100%) |
| E. coli HB101 (pCX311) | Alkaline phosphatase | 0.30 (60%) | 0.20 (40%) | 0.50 (100%) |
| | Penicillinase | 6.25 (77%) | 1.91 (23%) | 8.16 (100%) |

EP 0 316 023 B1

Example 5

Effects of inorganic salts in the media on xylanase production by Escherichia coli HB101 (pCX311) (FERM BP-470) were examined. The same medium as that used in Example 3 was used as a basic medium. The microorganism was cultured for 14 or 20 hours in the basic medium to which various inorganic salts had been added. Results are given in Table 2.

T a b l e  2

| Time (hrs.) | In- organic salt | Concent- ration of inorganic salt (M) | Xylanase activity (U/ml) | | |
|---|---|---|---|---|---|
| | | | Extracellular | Intracellular | Total |
| 14 | none | – | 0 | 0 | 0 |
| | NaCl | 0.08 | 0.07 | 0.11 | 0.18 |
| | " | 0.16* | 0.21 | 0.10 | 0.31 |
| | " | 0.32 | 0.38 | 0.10 | 0.48 |
| | " | 0.48 | 0.15 | 0.10 | 0.25 |
| | KCl | 0.16 | 0.25 | 0.12 | 0.37 |
| 20 | none | – | 0 | 0 | 0 |
| | NaCl | 0.08 | 0.06 | 0.07 | 0.13 |
| | " | 0.16 | 0.28 | 0.04 | 0.32 |
| | " | 0.32 | 0.40 | 0.13 | 0.53 |
| | " | 0.48 | 0.25 | 0.10 | 0.35 |
| | KCl | 0.16 | 0.28 | 0.02 | 0.30 |

\* 0.16M NaCl corresponds to 1% by weight.

Claims

1. A recombinant plasmid which is capable of inducing the extracellular secretion of xylanase in Escherichia coli transformed with said plasmid and which is obtainable by:
   (a) preparing with a restriction enzyme a chromosomal DNA fragment of Bacillus sp. C 125 (FERM BP-469) which codes for the extracellular secretion of xylanase;
   (b) preparing the recombinant plasmid DNA by ligating the chromosomal DNA fragment obtained in step (a) into a vector plasmid which was digested with a suitable restriction enzyme; and
   (c) isolating the recombinant plasmid DNA from a transformed host cell which exhibits a xylanase-producing phenotype.

11

**2.** The recombinant plasmid of claim 1, wherein the restriction enzyme used in steps (a) and (b) is the restriction enzyme Hind III.

**3.** The recombinant plasmid of Claim 1 or 2, wherein the vector plasmid is plasmid pBR 322.

**4.** The recombinant plasmid according to any one of Claims 1 to 3, which is the plasmid pCX 311 (FERM BP-470).

**5.** A host organism of the genus Escherichia, preferably of the species Escherichia coli, which is transformed with a recombinant plasmid according to any one of Claims 1 to 4.

**6.** A host organism according to Claim 5 which is the strain Escherichia coli HB101 and which is capable of extracellular secretion of xylanase.

**7.** A method for the construction of a recombinant plasmid according to Claim 1, which comprises the steps of:
(a) preparing with a restriction enzyme a chromosomal DNA fragment of Bacillus sp. C 125 (FERM BP-469) which codes for the extracellular secretion of xylanase;
(b) preparing the recombinant plasmid DNA by ligating the chromosomal DNA fragment obtained in step (a) into a vector which was digested with a suitable restriction enzyme; and
(c) isolating the recombinant plasmid DNA from a transformed host cell which exhibits a xylanase-producing phenotype.

**8.** The method of Claim 7, wherein the restriction enzyme used in steps (a) and (b) is the restriction enzyme Hind III.

**9.** The method of Claims 7 or 8, wherein the vector plasmid is pBR 322.

**10.** The method according to any one of Claims 7 to 9, wherein the recombinant plasmid is plasmid pCX 311 (FERM BP-470).

**11.** A method for the extracellular production of xylanase in which a transformant according to Claim 5 or 6 is cultivated and which comprises:
(a) inoculating a medium containing a selected carbon source together with an inorganic salt which is a sodium salt or a potassium salt with the transformant;
(b) keeping on culturing the transformant after the concentration of the transformant cells reaches the maximum and until the production and accumulation of xylanase in the medium reaches the maximum.

**12.** The method of Claim 11, wherein the inorganic salt is sodium chloride or potassium chloride.

**13.** The method of Claim 11 or 12, wherein the inorganic salt is used in an amount of 0.5 to 3.0 % by weight of the medium.

**14.** The method of any one of Claims 11 to 13, wherein the carbon source is bran or xylan.

**15.** The method of any one of Claims 11 to 14, wherein the transformant is cultured for 12 to 48 hours.

**Patentansprüche**

**1.** Rekombinantes Plasmid, das die extrazelluläre Sekretion von Xylanase in mit diesem Plasmid transformiertem Escherichia coli induzieren kann und das durch folgende Schritte erhältlich ist:
(a) Herstellung eines chromosomalen DNA-Fragments von Bacillus sp. C 125 (FERM BP-469), das die extrazelluläre Sekretion von Xylanase codiert, mit Hilfe eines Restriktionsenzyms;
(b) Herstellung der rekombinanten Plasmid-DNA durch Ligierung des in Schritt (a) erhaltenen chromosomalen DNA-Fragments in ein Vektorplasmid, das mit einem geeigneten Restriktionsenzym gespalten wurde; und

(c) Isolierung der rekombinanten Plasmid-DNA aus einer transformierten Wirtszelle, die einen Xylanase-produzierenden Phenotyp zeigt.

2. Rekombinantes Plasmid nach Anspruch 1, wobei das in den Schritten (a) und (b) verwendete Restriktionsenzym das Restriktionsenzym HindIII ist.

3. Rekombinantes Plasmid nach Anspruch 1 oder 2, wobei das Vektorplasmid das Plasmid pBR 322 ist.

4. Rekombinantes Plasmid nach einem der Ansprüche 1 bis 3, nämlich das Plasmid pCX 311 (FERM BP-470).

5. Wirtsorganismus der Gattung Escherichia, vorzugsweise der Art Escherichia coli, der mit einem rekombinanten Plasmid nach einem der Ansprüche 1 bis 4 transformiert ist.

6. Wirtsorganismus nach Anspruch 5, nämlich der Stamm Escherichia coli HB101, der zur extrazellulären Sekretion von Xylanase fähig ist.

7. Verfahren zur Konstruktion eines rekombinanten Plasmids nach Anspruch 1, umfassend die folgenden Schritte:
(a) Herstellung eines chromosomalen DNA-Fragments von Bacillus sp. C 125 (FERM BP-469), das die extrazelluläre Sekretion von Xylanase codiert, mit Hilfe eines Restriktionsenzyms,
(b) Herstellung der rekombinanten Plasmid-DNA durch Ligierung des in Schritt (a) erhaltenen chromosomalen DNA-Fragments in einen Vektor, der mit einem geeigneten Restriktionsenzym gespalten wurde;
(c) Isolierung der rekombinanten Plasmid-DNA aus einer transformierten Wirtszelle, die einen Xylanase-produzierenden Phenotyp zeigt.

8. Verfahren nach Anspruch 7, wobei das in den Schritten (a) und (b) verwendete Restriktionsenzym das Restriktionsenzym HindIII ist.

9. Verfahren nach den Ansprüchen 7 oder 8, wobei das Vektorplasmid pBR 322 ist.

10. Verfahren nach einem der Ansprüche 7 bis 9, wobei das rekombinante Plasmid das Plasmid pCX 311 (FERM BP-470) ist.

11. Verfahren zur extrazellulären Produktion von Xylanase, in dem ein Transformant nach Anspruch 5 oder 6 gezüchtet wird, umfassend:
(a) Inoculierung eines Mediums, das eine ausgewählte Kohlenstoffquelle zusammen mit einem anorganischen Salz, nämlich einem Natriumsalz oder einem Kaliumsalz, enthält, mit dem Transformanten;
(b) Weiterzüchtung des Transformanten, nachdem die Konzentration der Transformantenzellen das Maximum erreicht hat, und bis die Produktion und Anreicherung von Xylanase im Medium das Maximum erreicht.

12. Verfahren nach Anspruch 11, wobei das anorganische Salz Natriumchlorid oder Kaliumchlorid ist.

13. Verfahren nach Anspruch 11 oder 12, wobei das anorganische Salz in einer Menge von 0,5 bis 3,0 Gew.-% des Mediums verwendet wird.

14. Verfahren nach einem der Ansprüche 11 bis 13, wobei die Kohlenstoffquelle Kleie oder Xylan ist.

15. Verfahren nach einem der Ansprüche 11 bis 14, wobei der Transformant 12 bis 48 Stunden gezüchtet wird.

**Revendications**

1. Plasmide recombinant qui peut induire la sécrétion extracellulaire de xylanase dans Escherichia coli transformé par ledit plasmide et qui est obtenable :

(a) en préparant avec une enzyme de restriction un fragment d'ADN chromosomique de Bacillus sp. C 125 (FERM BP-469) qui code pour la sécrétion extracellulaire de xylanase;

(b) en préparant l'ADN de plasmide recombinant par ligation du fragment d'ADN chromosomique obtenu dans l'étape (a) dans un plasmide vecteur qui a été digéré avec une enzyme de restriction appropriée; et

(c) en isolant l'ADN de plasmide recombinant d'une cellule hôte transformée qui montre un phénotype produisant de la xylanase.

2. Plasmide recombinant suivant la revendication 1, caractérisé en ce que l'enzyme de restriction utilisée dans les étapes (a) et (b) est l'enzyme de restriction Hind III.

3. Plasmide recombinant suivant l'une ou l'autre des revendications 1 et 2, caractérisé en ce que le plasmide vecteur est le plasmide pBR 322.

4. Plasmide recombinant suivant l'une quelconque des revendications 1 à 3, qui est le plasmide pCX 311 (FERM BP-470).

5. Organisme hôte du genre Escherichia, de préférence de l'espèce Escherichia coli, qui est transformé par un plasmide recombinant suivant l'une quelconque des revendications 1 à 4.

6. Organisme hôte suivant la revendication 5, qui est la souche d'Escherichia coli HB101 et qui peut induire la sécrétion extracellulaire de xylanase.

7. Procédé pour la construction d'un plasmide recombinant suivant la revendication 1, qui comprend les étapes suivantes :

(a) la préparation avec une enzyme de restriction d'un fragment d'ADN chromosomique de Bacillus sp. C 125 (FERM BP-469) qui code pour la sécrétion extracellulaire de xylanase;

(b) la préparation de l'ADN de plasmide recombinant par ligation du fragment d'ADN chromosomique obtenu dans l'étape (a) dans un vecteur qui a été digéré avec une enzyme de restriction appropriée; et

(c) l'isolement de l'ADN de plasmide recombinant d'une cellule hôte transformée qui montre un phénotype produisant de la xylanase.

8. Procédé suivant la revendication 7, caractérisé en ce que l'enzyme de restriction utilisée dans les étapes (a) et (b) est l'enzyme de restriction Hind III.

9. Procédé suivant l'une ou l'autre des revendications 7 et 8, caractérisé en ce que le plasmide vecteur est le pBR 322.

10. Procédé suivant l'une quelconque des revendications 7 à 9, caractérisé en ce que le plasmide recombinant est le plasmide pCX 311 (FERM BP-470).

11. Procédé pour la production extracellulaire de xylanase dans lequel un transformant suivant l'une ou l'autre des revendications 5 et 6 est cultivé et qui comprend :

(a) l'inoculation d'un milieu contenant une source de carbone choisie ainsi qu'un sel inorganique qui est un sel de sodium ou un sel de potassium avec le transformant;

(b) le maintien en culture du transformant après que la concentration des cellules de transformant atteint le maximum et jusqu'à ce que la production et l'accumulation de xylanase dans le milieu atteignent le maximum.

12. Procédé suivant la revendication 11, caractérisé en ce que le sel inorganique est du chlorure de sodium ou du chlorure de potassium.

13. Procédé suivant l'une ou l'autre des revendications 11 et 12, caractérisé en ce que le sel inorganique est utilisé à raison de 0,5 à 3,0 % en poids du milieu.

14. Procédé suivant l'une quelconque des revendications 11 à 13, caractérisé en ce que la source de carbone est du son ou du xylane.

**EP 0 316 023 B1**

**15.** Procédé suivant l'une quelconque des revendications 11 à 14, caractérisé en ce que le transformant est cultivé pendant 12 à 48 heures.

15

# FIG. 1

XYLANASE ACTIVITY (UNIT/mℓ)

0.5 0.25 0

CULTURE TIME (hr)

0 5 10 15 20 48

ABSORBANCE AT 660 nm

5 4 3 2 1 0

o : EXTRACELLULAR
▲ : INTRACELLULAR
× : TOTAL ACTIVITY
● : GROWTH

# FIG. 2

○ : EXTRACELLULAR
▲ : INTRACELLULAR
× : TOTAL ACTIVITY
● : GROWTH

XYLANASE ACTIVITY (UNIT/mℓ)

ABSORBANCE AT 660nm

CULTURE TIME (hr)

# FIG. 3

# FIG. 4

Hind III

pCX 311
(9.01 kb)

—Hind III

Hind III

——————— : pBR 322 PLASMID DNA

: BACILLUS C125 DNA FRAGMENT
(XYLANASE DNA FRAGMENT)

# FIG. 5

○——○ E.coli HB 101 (pCX 311)
●----● BACILLUS C125